# EUROPEAN PATENT SPECIFICATION

(11) **EP 2 906 259 B1**
(45) Date of publication and mention of the grant of the patent: **01.05.2019**
(21) Application number: 13844598.6
(22) Date of filing: 18.09.2013
(51) Int. Cl.: G01N 33/94, A61K 47/10

(54) **MARKERS FOR PHARMACEUTICALS**
MARKER FÜR ARZNEIMITTEL
MARQUEURS POUR PRODUITS PHARMACEUTIQUES

(30) Priority: 10.10.2012 US 201261712099 P; 27.06.2013 US 201361840253 P
(43) Date of publication of application: 19.08.2015
(73) Proprietor: Keller, Ruprecht, 53937 Schleiden (DE)
(72) Inventor: Keller, Ruprecht, 53937 Schleiden (DE)
(74) Representative: K&L Gates LLP
(86) International application number: PCT/US2013/060427
(87) International publication number: WO 2014/058582

(56) References cited:
- WO-A1-02/075307
- WO-A2-2004/046715
- WO-A2-2011/032584
- US-A- 5 643 728
- US-A1- 2007 298 502
- US-A1- 2009 075 261
- US-A1- 2010 006 750
- US-A1- 2011 217 243
- US-A1- 2011 299 081
- US-B1- 6 440 667
- US-B2- 7 115 301
- BERND HUPPERTZ ET AL: "Urine labeling with orally applied marker substances in drug substitution therapy", CLINICAL CHEMISTRY AND LABORATORY MEDICINE, vol. 42, no. 6, 7 January 2004 (2004-01-07), pages 621-626, XP055282632, DE ISSN: 1434-6621, DOI: 10.1515/CCLM.2004.107
- GAUCHEL G ET AL: "Clinical use of polyethylene glycols as marker substances and determination in urine by liquid chromatography", JOURNAL OF CHROMATOGRAPHY B: BIOMEDICAL SCIENCES & APPLICATIONS, ELSEVIER, AMSTERDAM, NL, vol. 787, no. 2, 25 April 2003 (2003-04-25), pages 271-279, XP004414376, ISSN: 1570-0232, DOI: 10.1016/S1570-0232(02)00925-X

## Description

The authentication and tracking of pharmaceutical products is becoming an increasing concern by governmental agencies, manufacturers, distributors, sellers, and consumers, for a variety of reasons. For example, there has been a recent proliferation of counterfeit medications (i.e., pharmaceuticals manufactured by an unauthorized manufacturer) which are made to appear similar or even identical to legitimate products and often sold on the so-called "black market" or integrated into the normal stream of commerce, being passed off as legitimate. Such counterfeit medications may be contaminated, contain the wrong or no active ingredient, or even have the correct active ingredient but at an incorrect dosage.

Manufacturers, distributors, and/or sellers have an interest in tracking the source of legitimate pharmaceutical products. As a result of regional or country specific regulatory, patent, or market conditions, some pharmaceuticals have significant price disparity between countries. In such instances, pharmaceutical products authorized or intended for sale by the manufacturer within one country (low-price) may find their way through the so-called "grey market" for sale in another (high-price) country at significantly reduced prices. These grey market sales of legitimate products result in the loss of revenue and profits available in that second country. Thus, there is a need to track legitimate pharmaceutical products to determine in which country or region that product was intended for sale, thereby combatting grey market traffic.

Tracking the source of pharmaceutical products is also useful in case of inadvertent contamination or other adulteration. Labeled pharmaceutical products may be tracked to determine the particular factory or even individual batch from which the contaminated or adulterated pharmaceutical product originated. This is particularly useful for dispensed products (e.g., prescription products) for which the original manufacturer's package may be unknown or unidentifiable at the time of contamination detection (i.e., when contamination is detected after the product has been dispensed to the consumer).

An object of the present invention is to provide a means of authenticating or determining the source of the pharmaceutical product in question. In this context there is no question that any analytical investigation of a sample is only meaningful if the results obtained in the investigation can be used to determine if a pharmaceutical product was made by a particular manufacturer (in the case of authenticating potential black market goods), determine what geographic market was intended for the pharmaceutical product (in the case of authenticating potential grey market goods), or determine a particular factory or specific batch of origin (in the case of determining the origin of a pharmaceutical product), in order to then initiate the correct response.
The object has been solved by the features of the independent claim.

Also disclosed herein is a labeled pharmaceutical product comprising:
a pharmaceutically active ingredient; and
a unique marker profile encoding one or more of the origin of the pharmaceutical product, the intended recipient of the pharmaceutical product, and a preselected characteristic of an intended recipient of the pharmaceutical product;
wherein said unique marker profile comprises one or more pharmaceutically inactive marker substances, and wherein said pharmaceutically inactive marker substances are not identifiable by sequencing.

As disclosed herein, the pharmaceutical product contains at least two, three, four, five, or more marker substances.

When polymers (i.e. polyethylene glycols) are used as marker substances, polymers of different molecular weights are used as individual (i.e., different) marker substances. None of the marker substances are identifiable by sequencing (e.g., none of the marker substances comprise a nucleic acid).

Marker substances are substances identifiable or quantifiable by enzymatic, immunological, spectrometric, or electrophoretic methods and/or by an instrumental analytical chemistry technique including, for example, mass spectrometry (e.g., Gas Chromatography/Mass Spectrometry (GC/MS), Gas Chromatography/Tandem Mass Spectrometry (GC/MS/MS), High Performance Liquid Chromatography/Mass Spectrometry (HPLC/MS), High Performance Liquid Chromatography/Tandem Mass Spectrometry (HPLC/MS/MS), High Performance Liquid Chromatography (HPLC), or Gas Chromatography (GC)).

The unique marker profile comprises two or more pharmaceutically inactive marker substances, and at least two of said pharmaceutically inactive marker substances are the same type of marker substances. Alternatively, at least two of said pharmaceutically inactive marker substances are different types of marker substances.

As described herein, the marker substances include a disaccharide, trisaccharide, tetrasaccharide, oligosaccharide, or polysaccharide. In other preferred embodiments, the marker substances include a polydisperse polyethylene glycol and/or a monodisperse polyethylene glycol. Marker profiles according the the invention comprise two or more monodisperse polyethylene glycols each with different molecular weights.

According to the invention, the unique marker profile indicates the origin of said pharmaceutical product (e.g., the country, region, or manufacturing site (production facility)), as described herein the intended recipient of said pharmaceutical product (e.g., patient population) and/or a preselected characteristic of an intended recipient of said pharmaceutical product (e.g., geographic region in which an intended recipient is located). For example, the unique marker profile may include marker substances which indicate an intended recipient is found within a particular geographic region. In another example, the unique marker profile may include marker substances which indicate an intended recipient is subject to review by a particular regulatory or other governmental agency.

According to the invention, the identification of the marker profile and its indication is based on the identity of the marker substances (i.e., the presence or absence of the marker substances), and the relative amounts of the marker substances (i.e., the ratios of the marker substances relative to each other in the pharmaceutical product), or the absolute amounts of the marker substances in the pharmaceutical product, or some combination thereof.

As used herein, the term "origin of a pharmaceutical product" is an absolute term denoting the location or facility where a pharmaceutical product was manufactured.

As used herein, the term "source of a pharmaceutical product" is a relative term denoting the supplier of a pharmaceutical product. In some instances, the source of a pharmaceutical product may be the same as the place of origin, i.e., the location or facility of origin. In other instances, pharmaceutical products may be shipped from their location or facility of origin to a recipient, who may then themselves become a source of the pharmaceutical product if they provide the pharmaceutical products to another party.

As used herein, the term "derivative" is to be understood as all subsequent products which arise as a result of an induced or naturally occurring chemical transformation of a substance. Derivatives of marker substances can arise in the organism of the subject (e.g., by metabolism of the marker substance), or in a sample by induced or naturally occurring chemical transformation.

As used herein, the term "quantity" is to be understood as the amount of a particular substance and may be used to describe either an absolute measure of a substance in a sample, or to describe the relative amount of a substance in a sample with respect to some other substance(s) in the sample. Likewise, the term "quantify" is to be understood as to determine an absolute measure of a substance in a sample, or determine the relative amount of a substance in a sample with respect to some other substance(s) in the sample.

### DETAILED DESCRIPTION

The following description and examples describe in detail exemplary embodiments of pharmaceutical compositions which allow for authentication and/or determination of source, and methods for using the same. It should be appreciated that there are numerous variations and modifications of the compositions and methods described herein that are encompassed by the present invention. Accordingly, the description of a certain exemplary embodiment should not be deemed to limit the scope of the present invention.

The inventions provides the use of two or more pharmaceutically inactive polyethylene glycols (PEGs), each of the PEGs having a different molecular weight, as a unique marker profile to label a pharmaceutical product comprising a pharmaceutically active ingredient, wherein the unique marker profile has been incorporated in the pharmaceutical product during manufacture; wherein said two or more pharmaceutically inactive PEGs are not identifiable by sequencing, and wherein the identities and absolute or relative amounts of said two or more pharmaceutically inactive PEGs of said unique marker profile are determined and indicate the source of origin of said pharmaceutical product, wherein the source of origin is the location or facility where the pharmaceutical product was manufactured or denotes the supplier of the pharmaceutical product or the recipient to whom the pharmaceutical product is shipped from its location or facility of origin and who becomes himself a source of the pharmaceutical product if he provides the pharmaceutical product to another party.

### Marker Substances

Any suitable marker substance or combination of marker substances may be used as described herein. product without affecting the pharmacological behavior or usefulness of the pharmaceutical product, while providing a unique label associated with a particular source or intended recipient.

Advantageous marker substances are characterized in that they are detectable by known and routine detection methods already established in chemical investigation laboratories, such as for example common methods of clinical analytical chemistry. Such marker substances may or may not be taken up by the body upon administration, and are preferably FDA recognized as acceptable inactive ingredients for pharmaceutical formulations.

Marker substances are

Polyethylene glycols (including monodisperse and polydisperse polyethylene glycols).

Using PEG as marker substance, the molecular weight thereof is preferably less than about 5000 Da, 4000 Da, 3000 Da, 2000 Da, 1,500 Da, 1000 Da, 900 Da, 800 Da, 700 Da, 600 Da, 500 Da, or 400 Da, and/or greater than about 100 Da, 150 Da, 200 Da, 250 Da, 300 Da, 400 Da, and 500 Da. Typically, the polymer has at least about 2, 3, 4, 5, 7, 10, 15, 20, 25, 30, 40, 50, 75, or 100 repeating monomeric units and/or not more than about 15, 25, 50, 75, 100, 150, 200, 250, or 500 repeating monomeric units.

According to the invention, where the marker substances include two or more polyethylene glycols, each polyethylene glycol should be distinguishable by chemical and/or physical analysis. Each polyethylene glycol in the marker substance should have a different molecular weight. In this way, the identity and quantity and/or determine the relative amounts of each polyethylene glycol in the combination of marker substances may be determined e.g., by various forms of mass spectrometry.

As described herein, the marker substances are substances which are not absorbed by a subject who has taken the pharmaceutical. Determination of the unique pharmaceutical marker may typically be conducted by analyzing a sample of the pharmaceutical product itself.

As described herein, the marker substances are substances which are absorbed by a subject who has taken the pharmaceutical, and which can be detected in one or more body fluids, such as urine, blood, plasma, serum or the like, of the subject. As described herein, the marker substances are not metabolized following uptake by the subject. The marker substances are metabolized to a derivative specifically attributable to the marker substance. As described herein, where the marker substances are absorbed by a subject who has taken the pharmaceutical and are either not metabolized or metabolized to a derivative specifically attributable to the marker substances, determination of the unique marker profile may be conducted by analyzing a sample of the pharmaceutical product itself, as above, or by analyzing a biological sample obtained from a person who has consumed or otherwise been administered the pharmaceutical product.

As described herein, if the marker substances are soluble in a liquid based pharmaceutical product, the normal taste of the liquid is not changed by their addition.

### Incorporation of the marker substance(s) into a pharmaceutical product

It is preferable to use two or more pharmaceutically inactive PEGs to label a particular pharmaceutical product in a way that allows for determination of origin and/or source with a relatively high degree of certainty. In some instances, this may involve adding combinations of 2, 3, 4, 5, or more marker substances, each at its own independent concentration. It is readily apparent that the more marker substances that are present, each at their own independent levels of concentration, the more unlikely a particular combination is to be used as excipient in the routine manufacture of a pharmaceutical product.

As used herein, the term "pharmaceutical product" is intended to generally encompass all delivery vehicles for administration of an active to a patient. The concept is not to be limited to route of administration. Exemplary solid form pharmaceutical products include tablets, capsuls, gelcaps, powders, granules, and the like. Exemplary liquid form pharmaceutical products include forms suitable for ocular, nasal, topical, or oral administration, and injectable formulations including those intended for administration by the intravenous, intramuscular, intrathecal, and subcutaneous routes.

When incorporated into solid-form pharmaceutical products, the maker substances may be incorporated into any component of the product. For example, the markers substances may be included in the main formulation as described above, and added as inactive ingredient(s). Alternatively, the marker substances may be included as part of any other component of the formulation, such as a coating or gel cap, if such is used in the product.

As described above, a number of different marker substances may be used. However, not all marker substances may be suitable for all possible pharmaceutical formulation forms. For example, marker substances suitable for inclusion in a tablet or capsule may be different than those that are suitable for inclusion in a liquid formulation. It is apparent to one of skill in the art which marker substances are suitable for particular pharmaceutical forms.

Once suitable marker substances have been selected, the marker substances are added to a formulation at the manufacturing site. These additions are typically included as inactive ingredients comprising a percentage (by weight) of the total formulation. The mechanics of these additions will vary according to the particular pharmaceutical formulation being labeled and the nature and amount of the marker substances being included, but will be readily determined by those of skill in the art.

### Methods of identifying the source of a pharmaceutical product

The number of marker substances and amount of each included in a pharmaceutical product may be varied to achieve any of the uses described herein. For example, each of plurality of manufacturing facilities may be assigned a unique marker profile (i.e., a unique set of marker substances, optionally including the absolute or relative concentrations of each marker substance) that is incorporated into pharmaceutical products manufactured at that facility. In this way, the particular manufacturing plant of any individual pharmaceutical product (i.e., a pharmaceutical product's place of origin) may later be identified by determining the identities (and possibly amounts) of marker substances contained in the pharmaceutical product and comparing this information to known marker profiles for each possible manufacturing facility. Such a determination facilitates authentication of a pharmaceutical product, and thus aids in identification of counterfeit pharmaceuticals.

In another embodiment, a larger number of unique marker profiles may be generated from a smaller number of marker substances by varying the relative concentrations of the marker substances in known/pre-determined ratios. For example, in its simplest form when only two marker substances are used, represented generically as X and X', multiple marker profiles may be constructed by varying the ratios of X to X' as follows:

| | Relative Amount of Marker Substance | |
|---|---|---|
| Marker # | X | X' |
| 1 | 1 | 0 |
| 2 | 0 | 1 |
| 3 | 1 | 1 |
| 4 | 1 | 2 |
| 5 | 1 | 3 |
| 6 | 1 | 4 |
| 7 | 2 | 1 |
| 8 | 3 | 1 |
| 9 | 4 | 1 |

The ratios of the paired marker substances are not limited to those ratios shown in the table above but may include any convenient ratio or combination of ratios such as 5:1, 10:1, 15:1, 20:1, or more. The only practical limits are those of convenience and detectability. Furthermore, the strategy of constructing unique marker profiles based on the relative ratios of marker substances is not limited to pairs of marker substances but instead can be extended to varying the relative ratios of 3, 4, 5, 6, 7, 8, or more marker substances, thereby significantly increasing the total number of unique marker profiles available for any given number of marker substances, without increasing the number of chemically distinct marker substances.

It should be understood that in some embodiments, the identity of a marker substance and the quantity of that marker substance may each be used to convey different information. The following example is not intended to be limiting and is intended to illustrate one possible embodiment. Take for example a scenario where some particular pharmaceutical is prepared in capsule form by a Manufacturing Facility 1 with two different Intended Recipients. Manufacturing Facility 1 may be assigned a unique marker profile comprising two marker substances, A and B. Thus, all capsules manufactured at Manufacturing Facility 1 include both marker substances A and B. However, the amounts (absolute or relative) of the two marker substances may be varied to indicate the intended recipient. For example, capsules manufactured for Intended Recipient 1 may comprise some amount of marker substance A, and twice that amount of marker substance B. Capsules manufactured for Intended Recipient 2 may comprise some amount of marker substance A, and one half that amount of marker substance B. In this way, later analysis of the identity of the marker substances allows for determination of the source of the pharmaceutical (i.e., Manufacturing Facility 1 because of the presence of both marker substances A and B), and analysis of the relative amounts of the marker substances allows for the determination of the intended recipient of the capsule.

As described herein, each piece of information that is to be conveyed by the marker substances is encoded with at least one different marker substance. The following illustrative example is provided to contrast the example described above. Consider the same scenario as above where some particular pharmaceutical is prepared in capsule form by Manufacturing Facility 1 with two different Intended Recipients. Manufacturing Facility 1 may be assigned a unique marker profile comprising two marker substances, A and B. Thus, all capsules manufactured at Manufacturing Facility 1 include both marker substances A and B. One or more additional marker substances are used to encode the identity of the intended recipients. For example, capsules manufactured for Intended Recipient 1 may comprise some amount of an additional marker substance C, while capsules manufactured for Intended Recipient 2 may comprise some amount of additional marker substances D and E. In this way, later analysis of the identity of the marker substances in a capsules from this facility would show the presence of marker substances A and B, and either C or D and E. The identification of marker substances A and B allows for determination of the source of the pharmaceutical (i.e., Manufacturing Facility 1), and identification of marker substances C or D and E allows for the determination of the intended recipient of the pharmaceutical.

The above illustrative examples demonstrate that a plurality of marker substances may be used. In fact, the above examples may be considered to be relatively simple, in as far as the number of manufacturing facilities and intended recipients in the examples are very limited. However, it should be appreciated that the number of marker substances that can be used in any given unique marker profile is only limited by the number of suitable marker substances available. It should also be appreciated that a unique marker profile may contain as few as a single marker substance, with or without consideration of its relative or absolute concentration in the pharmaceutical product.

It is especially preferable to include multiple marker substances in a pharmaceutical product, wherein it is possible by the combination of marker substances to develop a certain numerical code belonging to a respective sample. For example, it is preferred to include a combination of at least 2, such as at least 3, such as at least 4, such as at least 5 marker substances simultaneously. Using a total of n marker substances, there exist 2ⁿ⁻¹ different combinations in a dual numeric system; that is without use of absolute or relative concentrations of each marker as additional indicia.

In some embodiments, a unique marker profile comprises a plurality of marker substances, and two or more of the plurality of marker substances are of the same type of marker substances (i.e. two or more polyethylene glycols, etc. as described elsewhere). As described herein, every marker substance in the plurality of marker substances are of the same type of marker substance.

As described herein, a unique marker profile comprises a plurality of marker substances, and two or more of the plurality of marker substances are different types of marker substances.

As described herein, a unique marker profile comprises three or more marker substances, and two or more marker substances are of the same type of marker substance, while at least one of the three or more is of a different type of marker substance.

As described above, at least one marker substance may not be absorbed by the body upon administration. Determination of the marker profile may be conducted by analyzing the pharmaceutical product itself. For example, if the pharmaceutical product is a capsule, at least a portion of the capsules may be crushed, dissolved in an appropriate solvent, and an aliquot of the resulting solution analyzed by the appropriate technique to identify and/or quantitate and/or determine the relative amounts of each marker substance present in the capsule. Once the unique marker profile has been identified, this information can be used to determine the site of origin and/or intended recipient or recipients of the capsule.

Also as described above, one or more marker substances may be substances which are absorbed by a subject who has taken the pharmaceutical product, and which can be detected in one or more body fluids, such as urine, blood, plasma, serum or the like, of the subject. As described herein, determination of the unique marker profile may be conducted by analyzing a sample of the pharmaceutical product itself, as above, or by analyzing a biological sample obtained from a person who has consumed or otherwise been administered the pharmaceutical product.

For example, consider a pharmaceutical liquid composition, such as a prescription cough syrup, that may be find use as an illicit drug. If a patient is confirmed to have illegally obtained and used the pharmaceutical liquid composition, an appropriate body fluid from the patient, such as a urine or blood sample, can be analyzed to identify the unique marker profile of the consumed cough syrup, so that it can be determined if the patient obtained the liquid composition from black market, grey market, or otherwise legitimate sources. This information may potentially assist law enforcement in tracking the illicit supplier of the pharmaceutical liquid composition.

As described herein, where a body fluid from a patient is to be analyzed for detection of the marker substances, any means of sample collection known to be suitable in the art can be used. For example, sample removal occurs in different ways depending on the type of sample to be investigated. In the case of the analysis of body excretions, part of the sample is taken up into a sample vessel and, after this time, is ready for further investigation. In the investigation of human urine or stool samples, the samples can usually be furnished by the subjects themselves in that the subject is simply given a sample vessel. For the removal of samples from body fluids or from tissue samples, a direct operation on the subject is normally necessary. Here, obtention of blood from the subject can be accomplished using a suction pipette following pricking or cutting of the skin with a disposable lancet or--in larger quantities--using an injection syringe or blood collection tube after puncture of the vein. For the investigation of liquor, the latter is obtained by lumbar, suboccipital or ventricle puncture.

By "biological sample" it is meant the components of a mammal designated for the analytical investigation. Relevant here are body excretions, body fluids or tissue samples. The components making up the sample can include components of a mammalian organism which still exist in the mammal at the time of sample removal as well as previous components of the mammal. By "body excretions" or "excretion" are to be understood urine, stool, secretions from salivary, milk, tear and sweat glands.

The term "body fluid" is to be understood as extracellular liquids of a mammalian organism (including male or female humans) like blood, serum and liquor. Preferably, the samples removed from or excreted by a mammal are body excretions, body fluids or tissue samples.

The term "tissue sample" is to be understood as an organization of identically differentiated cells obtained by a direct operation into the living mammalian organism, as well as these cells' intercellular substance. Hair samples and samples of sloughed-off parts of skin are also to be understood as falling within the meaning of this term.

Depending on the type of the sample and the at least one marker substance to be detected, the respective sample may have to be prepared prior to the analysis method. The preparation steps can include centrifugation for the separation of solid, non-solubilized materials in a liquid sample, solubilization or suspension of solid samples, concentration, precipitation with suitable reagents such as ammonium sulfate, adjustment of the pH value required for the analysis method, homogenization of the sample such as by ultrasonication or by using vibration cell mills, separation of materials used in lysing the sample such as for example detergents and other preparation steps known to one of ordinary skill in the art.

A number of enzymatic, immunological, mass spectrometric and electrophoretic detection methods as well as combinations of these methods are available for determining the identity and quantity and/or relative amounts of at least one marker substance in a sample. Preferably, analysis is conducted by an instrumental analytical chemistry technique, such as coupled Gas Chromatography/Mass Spectrometry (GC/MS) (with single or tandem MS), High Performance Liquid Chromatography/Mass Spectrometry (HPLC/MS) (with single or tandem MS), High Performance Liquid Chromatography (HPLC), or Gas Chromatography (GC). These methods allow the very time-efficient investigation of, in particular, liquid samples or of samples which, due to their preparation were transferred into a liquid. At the same time, these detection methods allow a high degree of automation so that a multitude of samples can be analyzed in a short time and, in as far the chromatograms and, as the case may be, mass spectroscopic fractionation patterns of reference substances already exist in the computer evaluation unit, the analysis of the at least one marker substance is also greatly simplified.

### EXAMPLE 1

As an example, an embodiment for uniquely marking of a liquid pharmaceutical product to enable later identification of the manufacturing facility of origin is provided below.

A particular liquid pharmaceutical product is manufactured at three manufacturing facilities: Facility 1, Facility 2, and Facility 3. Each manufacturing facility is assigned a unique marker profile comprising two marker substances. These two marker substances are added in equal amounts during the manufacture of the liquid pharmaceutical, such that the total concentration of the marker substances is about 1% of the liquid pharmaceutical.

At Facility 1, the liquid pharmaceutical product is produced such that the product comprises about 0.5%wt of Marker A, a monodisperse polyethylene glycol with an approximate molecular weight of about 530 amu, and about 0.5%wt of Marker B, a monodisperse polyethylene glycol with an approximate molecular weight of about 574 amu.

At Facility 2, the liquid pharmaceutical product is produced such that the product comprises about 0.5%wt of Marker A, and about 0.5%wt of Marker C, a monodisperse polyethylene glycol with an approximate molecular weight of about 618 amu.

At Facility 3, the liquid pharmaceutical product is produced such that the product comprises about 0.5%wt of Marker B, and about 0.5%wt of Marker C.

After production, the products are distributed to wholesale suppliers, and ultimately to approved retail locations, where the products are available for purchase by patients with an appropriate prescription. If a liquid pharmaceutical product purported to be genuine is later found at an unapproved retail location, the responsible law enforcement or regulatory body may send a sample of the purportedly genuine liquid pharmaceutical product for mass spectrometric analysis for identification and quantitation of marker substances present in the product.

Several possible results of such analysis, and their meanings with respect to origin are presented below in Table 1.

**Table 1**

| **Possible Outcome of Mass Spectrometric Analysis** | **Origin** |
|---|---|
| Markers A and B, at about 1:1 | Facility 1 |
| Markers A and C, at about 1:1 | Facility 2 |
| Markers B and C, at about 1:1 | Facility 3 |
| No Markers present | Unknown (counterfeit) |
| Single Marker present | Unknown (counterfeit) |
| Two Markers present, but not at about 1:1 | Unknown (counterfeit) |
| Three or more Markers present | Unknown (counterfeit) |

### EXAMPLE 2

As an example, an embodiment for uniquely marking of a liquid pharmaceutical product to enable later identification of the manufacturing facility of origin, and intended recipient is provided below.

A particular liquid pharmaceutical product is manufactured at three manufacturing facilities: Facility 1 and Facility 2. There are two intended recipients for the pharmaceutical products, and each intended recipient may receive pharmaceutical products from either Facility. The intended recipients for the product are Intended Recipient 1 and Intended Recipient 2.

Each combination of manufacturing facility and intended recipient is assigned a unique marker profile comprising four marker substances. Two marker substances are added in equal amounts during the manufacture of the liquid pharmaceutical, such that the total concentration of these two marker substances is about 1% of the liquid pharmaceutical. Two other marker substances are added at a ratio of 2:1 during the manufacture of the liquid pharmaceutical, such that the total concentration of these marker substances is about 1.5% of the liquid pharmaceutical. Thus, the total concentration of the four marker substances is about 2.5% of the total liquid pharmaceutical.

At Facility 1, all liquid pharmaceutical products, regardless of intended recipient, are produced such that the product comprises about 0.5%wt of Marker A, a monodisperse polyethylene glycol with an approximate molecular weight of about 530 amu, and about 0.5%wt of Marker B, a monodisperse polyethylene glycol with an approximate molecular weight of about 574 amu.

At Facility 2, all liquid pharmaceutical products, regardless of intended recipient, are produced such that the product comprises about 0.5%wt of Marker A, and about 0.5%wt of Marker C, a monodisperse polyethylene glycol with an approximate molecular weight of about 618 amu.

All liquid pharmaceutical products intended for Intended Recipient 1, regardless of production facility, are produced such that the product comprises about 0.5%wt of Marker D, a monodisperse polyethylene glycol with an approximate molecular weight of about 662 amu, and about 1%wt of Marker E, a monodisperse polyethylene glycol with an approximate molecular weight of about 706 amu.

All liquid pharmaceutical products intended for Intended Recipient 1, regardless of production facility, are produced such that the product comprises about 1%wt of Marker D, and about 0.5%wt of Marker E.

After production, the products are distributed to wholesale suppliers, and ultimately to approved retail locations, where the products are available for purchase by patients with an appropriate prescription. If a liquid pharmaceutical product purported to be genuine is later found at an unapproved retail location, the responsible law enforcement or regulatory body may send a sample of the purportedly genuine liquid pharmaceutical product for mass spectrometric analysis.

Several possible results of such analysis, and their meanings with respect to origin and intended recipient are presented below in Table 2.

**Table 2**

| **Possible Outcome of Mass Spectrometric Analysis** | **Origin** | **Intended Recipient** |
|---|---|---|
| Markers A, B, D, E; present at about 1:1:1:2 | Facility 1 | Intended Recipient 1 |
| Markers A, B, D, E; present at about 1:1:2:1 | Facility 1 | Intended Recipient 2 |
| Markers A, C, D, E; present at about 1:1:1:2 | Facility 2 | Intended Recipient 1 |
| Markers A, C, D, E; present at about 1:1:2:1 | Facility 2 | Intended Recipient 2 |
| No Markers present | Unknown (counterfeit) | N/A |
| Single Marker present | Unknown (counterfeit) | N/A |
| Two Markers present | Unknown (counterfeit) | N/A |
| Three Markers present | Unknown (counterfeit) | N/A |
| Four Markers present, but wrong combination | Unknown (counterfeit) | N/A |
| Four Markers present, but at incorrect ratio | Unknown (counterfeit) | N/A |
| Five or more Markers present | Unknown (counterfeit) | N/A |

If the pharmaceutical product under investigation is found to lack Markers A and B, or A and C, then the pharmaceutical product is known to be counterfeit.

If the pharmaceutical product is validated as genuine as to source but is found on the black or grey market, knowledge of the intended recipient, and thus the intended distribution network through which that product was intended to proceed, may prove useful in investigating the break in the supply chain which lead to availability of the pharmaceutical product on the black or grey market.

## Claims

1. The use of two or more pharmaceutically inactive polyethylene glycols (PEGs), each of the PEGs having different molecular weight, as a unique marker profile to label a pharmaceutical product comprising a pharmaceutically active ingredient, wherein the unique marker profile has been incorporated in the pharmaceutical product during manufacture; wherein said pharmaceutically inactive PEGs are not identifiable by sequencing, wherein the identities and absolute or relative amounts of said two or more pharmaceutically inactive PEGs of said unique marker profile are determined and indicate the source of origin of said pharmaceutical product,
wherein the source of origin is the location or facility where the pharmaceutical product was manufactured
or denotes the supplier of the pharmaceutical product or the recipient to whom the pharmaceutical product is shipped from its location or facility of origin and who becomes himself a source of the pharmaceutical product if he provides the pharmaceutical product to another party.

2. The use of claim 1, wherein said pharmaceutically inactive PEGs are identifiable or quantifiable by an instrumental analytical chemistry technique, or by mass spectrometry, or by Gas Chromatography/Mass Spectrometry (GC/MS), Gas Chromatography/Tandem Mass Spectrometry (GC/MS/MS), High Performance Liquid Chromatography/Mass Spectrometry (HPLC/MS), High Performance Liquid Chromatography/Tandem Mass Spectrometry (HPLC/MS/MS), High Performance Liquid Chromatography (HPLC), or Gas Chromatography (GC).

3. The use of claim 1, wherein at least one of said pharmaceutically inactive PEGs is a polydisperse polyethylene glycol, or a monodisperse polyethylene glycol.

4. The use of claim 1, wherein said PEGs comprise two or more monodisperse polyethylene glycols each with different molecular weight.

## Patentansprüche

1. Verwendung von zwei oder mehreren pharmazeutisch inaktiven Polyethylenglykolen (PEGs), wobei jedes der PEGs ein unterschiedliches Molekulargewicht als einzigartiges Markerprofil aufweist, um ein pharmazeutisches Produkt zu markieren, das einen aktiven Inhaltsstoff umfasst, wobei das einzigartige Markerprofil in das pharmazeutische Produkt während der Herstellung aufgenommen wurde;
wobei die pharmazeutisch inaktiven PEGs durch Sequenzierung nicht identifizierbar sind,
wobei die Identitäten und die absoluten oder relativen Mengen der zwei oder mehreren pharmazeutisch inaktiven PEGs des eindeutigen Markerprofils bestimmt werden und die Herkunftsquelle des pharmazeutischen Produkts angeben,
wobei die Herkunftsquelle der Standort oder die Einrichtung ist, an dem das pharmazeutische Produkt hergestellt wurde,
oder den Lieferanten des pharmazeutischen Produkts oder des Empfängers angibt, an den das pharmazeutische Produkt von seinem Standort oder seiner Herkunftsstätte aus versandt wird und der selbst eine Quelle des pharmazeutischen Produkts wird, wenn er das pharmazeutische Produkt einer anderen Partei zur Verfügung stellt.

2. Verwendung nach Anspruch 1, wobei die pharmazeutisch inaktiven PEGs durch eine instrumentelle analytische Chemietechnik oder durch Massenspektrometrie oder durch Gaschromatographie / Massenspektrometrie (GC/MS), Gaschromatographie / Tandem-Massenspektrometrie (GC/MS/ MS), Hochleistungsflüssigchromatographie / Massenspektrometrie (HPLC/MS), Hochleistungsflüssigchromatographie / Tandem-Massenspektrometrie (HPLC/MS/MS), Hochleistungsflüssigkeitschromatographie (HPLC) oder Gaschromatographie (GC) identifizierbar und quantifizierbar sind.

3. Verwendung nach Anspruch 1, wobei mindestens eines der pharmazeutisch inaktiven PEGs ein polydisperses Polyethylenglycol oder ein monodisperses Polyethylenglycol ist.

4. Verwendung nach Anspruch 1, wobei die PEGs zwei oder mehr monodisperse Polyethylenglykole mit jeweils unterschiedlichem Molekulargewicht umfassen.

## Revendications

1. Utilisation de deux polyéthylènes glycols (PEG) pharmaceutiquement inactifs ou plus, chacun des PEG ayant un poids moléculaire différent, en tant que profil de marqueur unique pour marquer un produit pharmaceutique comprenant un principe pharmaceutiquement actif, dans laquelle le profil de marqueur unique a été incorporé dans le produit pharmaceutique pendant la fabrication ;
dans laquelle lesdits PEG pharmaceutiquement inactifs ne sont pas identifiables par séquençage,
dans laquelle les identités et quantités absolues ou relatives desdits deux PEG pharmaceutiquement inactifs ou plus dudit profil de marqueur unique sont déterminées et indiquent la source d'origine dudit produit pharmaceutique,
dans laquelle la source d'origine est l'emplacement ou le site sur lequel le produit pharmaceutique a été fabriqué
ou désigne le fournisseur du produit pharmaceutique ou le destinataire à qui le produit pharmaceutique est expédié depuis son emplacement ou site d'origine et qui devient lui-même une source du produit pharmaceutique s'il fournit le produit pharmaceutique à un tiers.

2. Utilisation selon la revendication 1, dans laquelle lesdits PEG pharmaceutiquement inactifs peuvent être identifiés ou quantifiés par une technique de chimie analytique instrumentale, ou par spectrométrie de masse, ou par chromatographie en phase gazeuse/spectrométrie de masse (GC/MS), chromatographie en phase gazeuse/spectrométrie de masse en tandem (GC/MS/MS), chromatographie liquide haute performance/spectrométrie de masse (HPLC/MS), chromatographie liquide haute performance/spectrométrie de masse en tandem (HPLC/MS/MS), chromatographie liquide haute performance (HPLC) ou chromatographie en phase gazeuse (GC).

3. Utilisation selon la revendication 1, dans laquelle au moins l'un desdits PEG pharmaceutiquement inactifs est un polyéthylène glycol polydispersé ou un polyéthylène glycol monodispersé.

4. Utilisation selon la revendication 1, dans laquelle lesdits PEG comprennent deux polyéthylènes glycols monodispersés ou plus chacun ayant un poids moléculaire différent.
